# FASCICULE DE BREVET EUROPEEN

(11) **EP 2 338 571 B1**
(45) Date de publication et mention de la délivrance du brevet: **10.05.2017**
(21) Numéro de dépôt: 10195230.7
(22) Date de dépôt: 15.12.2010
(51) Int. Cl.: A61Q 5/08, A61Q 5/10, A61K 8/22, A61K 8/31, A61K 8/34, A61K 8/41, A61K 8/67

(54) **Agent de coloration et/ou de décoloration des fibres kératiniques en deux parties, comprenant un corps gras particulier et une réductone**
Substanz zum Färben und/oder Entfärben von Keratinfasern bestehend aus zwei Komponenten, die einen speziellen Fettkörper und eine Redukton enthält
Agent for colouring and/or bleaching keratinous fibres in two parts, including a particular fatty body and a reductone

(30) Priorité: 22.12.2009 FR 0959434
(43) Date de publication de la demande: 29.06.2011
(73) Titulaire: L'Oréal, 75008 Paris (FR)
(72) Inventeur: Goget, Caroline, 75018, Paris (FR); Deconinck, Gautier, 95210, Saint-Gratien (FR)
(74) Mandataire: Dossmann, Gérard

(56) Documents cités:
- EP-A1- 2 047 841
- EP-A1- 2 072 034
- EP-A1- 2 198 831
- EP-A1- 2 198 843
- EP-A1- 2 198 849
- EP-A2- 1 813 254
- EP-A2- 2 198 842
- FR-A- 1 338 063
- FR-A1- 2 925 308

## Description

La présente invention concerne un agent en deux parties, destiné à la coloration des fibres kératiniques, en particulier des fibres kératiniques humaines telles que les cheveux.

Plus précisément, la présente invention a pour objet un agent de coloration des fibres kératiniques, constitué d'une première composition (A) contenant un ou plusieurs agents alcalinisants et un ou plusieurs colorants, et d'une seconde composition (B) contenant un ou plusieurs agents oxydants, l'une au moins des deux compositions (A) et (B) comprenant un ou plusieurs corps gras particuliers, et une ou plusieurs réductones.

La présente invention concerne également un dispositif à plusieurs compartiments, contenant l'agent de coloration selon l'invention.

La présente invention a enfin pour objet un procédé de coloration des fibres kératiniques, mettant en oeuvre l'agent selon l'invention.

Depuis longtemps, de nombreuses personnes cherchent à modifier la couleur de leurs cheveux, et notamment à les colorer par exemple afin de masquer leurs cheveux blancs.

Pour colorer les fibres kératiniques humaines, essentiellement deux types de colorations ont été développés.

Le premier type de coloration est la coloration dite permanente ou d'oxydation, qui met en oeuvre des compositions tinctoriales contenant des précurseurs de colorant d'oxydation, appelés généralement bases d'oxydation. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration. La variété des molécules mises en jeu au niveau des bases d'oxydation et des coupleurs permet l'obtention d'une riche palette de couleurs.

Le deuxième type de coloration est la coloration dite semi-permanente ou coloration directe, qui consiste à appliquer sur les fibres kératiniques des colorants directs qui sont des molécules colorées et colorantes ayant une affinité pour lesdites fibres, à laisser poser, puis à les rincer.

Afin de réaliser ces colorations, les colorants directs généralement employés sont choisis parmi les colorants directs nitrés benzéniques, anthraquinoniques, nitropyridiniques, azoïques, xanthéniques, acridiniques, aziniques ou triarylméthaniques.

Ce type de procédé ne nécessite pas l'emploi d'un agent oxydant pour développer la coloration. Cependant, il est possible de mettre en oeuvre un tel agent, afin d'obtenir un effet d'éclaircissement avec la coloration. On parle alors d'une coloration directe ou semi-permanente en conditions éclaircissantes.

Les procédés de coloration permanente ou semi-permanente en conditions éclaircissantes nécessitent donc d'employer, avec la composition tinctoriale, une composition aqueuse comprenant au moins un agent oxydant, en condition de pH alcalin dans la grande majorité des cas.

L'une des difficultés rencontrées lors de la mise en oeuvre des procédés de coloration de l'art antérieur vient du fait qu'ils sont mis en oeuvre dans des conditions alcalines. [A]

Afin d'améliorer les performances des procédés de coloration des fibres kératiniques humaines, et de limiter les désagréments liés à l'emploi d'agents alcalins et d'agents oxydants, il a été proposé d'employer dans les compositions tinctoriales une quantité substantielle d'un ou plusieurs corps gras.

Cependant, lors du mélange, enrichi en corps gras, d'une composition comprenant un agent alcalin et d'une composition comprenant un agent oxydant, il se produit une coloration intense du mélange qui, d'une part, est très inesthétique, et, d'autre part, traduit une dégradation indésirable de certains composés.

La Demanderesse a maintenant découvert que de certains agents antioxydants particuliers appartenant à la famille des réductones, en présence de corps gras particuliers choisis parmi les hydrocarbures liquides, permettait de réduire fortement ce phénomène de coloration du mélange.

La présente invention a donc pour objet un agent de coloration des fibres kératiniques, constitué par:
- une première composition (A) comprenant un ou plusieurs agents alcalinisants, un ou plusieurs colorants d'oxydation, choisis parmi les bases d'oxydation, éventuellement combinée(s) à un ou plusieurs coupleurs, et un ou plusieurs colorants directs, et
- une seconde composition (B) comprenant un ou plusieurs agents oxydants,
l'une au moins des deux compositions (A) et (B) comprenant un ou plusieurs corps gras choisis parmi les hydrocarbures liquides, la quantité totale desdits hydrocarbures liquides dans le mélange des compositions (A) et (B) représentant au moins 20% en poids, par rapport au poids total dudit mélange, et
l'une au moins des deux compositions (A) et (B) comprenant une ou plusieurs réductones.
[A] = Par ailleurs, il est connu que l'acide ascorbique peut être utilisé en tant qu'agent réducteur dans des teintures pour cheveux contenant des colorants d'oxydation, comme l'indique le document FR 1 338 063.

Lorsque l'agent selon l'invention est destiné à la coloration, éventuellement éclaircissante, des fibres kératiniques, la composition (A) comprend en outre un ou plusieurs colorants d'oxydation et/ou un ou plusieurs colorants directs.

L'agent de coloration selon la présente invention ne se colore pas ni lors du mélange des deux compositions (A) et (B), ni lors de l'application séquentielle de ces deux compositions sur les fibres kératiniques, ou, si un phénomène de coloration se produit, celui-ci reste très modéré.

En outre, l'agent selon l'invention est particulièrement efficace sur le plan de la puissance de la coloration obtenue, ainsi que sur celui de la chromaticité, et de la sélectivité de la coloration d'une même fibre, ou entre des fibres différemment sensibilisées.

L'agent selon l'invention présente également l'avantage de limiter les odeurs agressives lors de sa préparation, ou de son application sur les fibres.

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine.

Les fibres kératiniques humaines traitées par le procédé selon l'invention sont de préférence les cheveux.

Selon la présente invention, la composition (A) comprend un ou plusieurs agents alcalinisants.

L'agent alcalinisant peut être en particulier une base minérale ou organique.

De préférence, l'agent alcalinisant est choisi parmi l'ammoniaque, les carbonates alcalins, les hydroxydes de sodium ou de potassium, les aminés organiques telles que par exemple les alcanolamines et leurs dérivés, et les composés de formule (I) suivante : 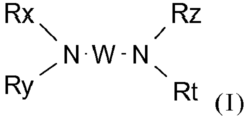 dans laquelle W est un reste alkylène en C₁-C₆ éventuellement substitué par un groupement hydroxyle ou un radical alkyle en C₁-C₆; Rx, Ry, Rz et Rt, identiques ou différents, représentent un atome d'hydrogène, un radical alkyle en C₁-C₆, hydroxyalkyle en C₁-C₆, ou aminoalkyle en C₁-C₆.

On peut citer à titre d'exemple de tels composés de formule (I) le 1,3 diaminopropane, le 1,3 diamino 2 propanol, la spermine, la spermidine.

Les agents alcalinisants particulièrement préférés sont les alcanolamines, et en particulier les mono-, di- et triéthanolamines.

Dans une variante préférée de l'invention, l'agent alcalinisant est la monoéthanolamine.

Selon un mode de réalisation particulier, la composition (A) contient en tant qu'agent alcalinisant au moins une aminé organique, de préférence au moins une alcanolamine. Lorsque la composition (A) contient plusieurs agents alcalinisants dont une alcanolamine et de l'ammoniaque ou un de ses sels, la ou les amines organiques sont de préférence majoritaires en poids, par rapport à la quantité d'ammoniac présent dans la composition (A).

Selon un mode de réalisation préféré de la présente invention, la composition (A) ne contient pas d'ammoniaque.

Selon un mode de réalisation également préféré de la présente invention, lorsque la composition (A) contient de l'ammoniaque ou un de ses sels, elle contient également une ou plusieurs alcanolamines, et la quantité pondérale d'alcanolamine(s) dans la composition (A) est supérieure à la quantité pondérale d'ammoniac dans cette même composition.

Généralement, la composition (A) présente une teneur en agent(s) alcalinisant(s) allant de 0,1 à 40 % en poids, de préférence de 0,5 à 20 % en poids, par rapport au poids de cette composition.

De préférence, la composition (A) présente un pH supérieur ou égal à 8, et plus préférentiellement un pH allant de 8,5 à 11,5.

Ce pH peut également être ajusté la valeur souhaitée par l'emploi, en plus de l'agent alcalinisants, d'un ou plusieurs agents acidifiants.

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Selon la présente invention, la composition (B) comprend un ou plusieurs agents oxydants,
Cet agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux comme le sodium, le potassium, le magnésium. On peut également utiliser à titre d'agent oxydant une ou plusieurs enzymes d'oxydo-réduction telles que les laccases, les peroxydases et les oxydoréductases à 2 électrons (telles que l'uricase), éventuellement en présence de leur donneur ou cofacteur respectif.

L'utilisation du peroxyde d'hydrogène est particulièrement préférée. Celui-ci peut être avantageusement employé en solution aqueuse (eau oxygénée) dont la concentration peut varier, plus particulièrement, de 0,1 à 50% en poids, et encore plus préférentiellement de 0,5 à 20% en poids, mieux de 1 à 15% en poids par rapport au poids total de la composition (B).

En fonction du degré de décoloration souhaité, l'agent oxydant peut également comprendre un ou plusieurs composés choisis de préférence parmi les sels peroxygénés.

De préférence, le pH de la composition (B) est inférieur à 7. Ce pH peut être ajusté à la valeur souhaitée par l'emploi d'un ou plusieurs agents acidifiants, qui peuvent être notamment choisis parmi ceux décrits précédemment.

Selon la présente invention, l'une et/ou l'autre des deux compositions (A) et (B) comprend une ou plusieurs réductones. Ainsi, la ou les réductones peuvent être présentes dans la composition (A), ou la composition (B), ou les deux compositions à la fois.

Avantageusement, la quantité de réductone(s) dans la composition (A) et/ou la composition (B) représente de 0,01 à 1% en poids, de préférence de 0,05 à 0,5% en poids, et mieux encore de 0,1 à 0,25% en poids, par rapport au poids total de la composition (A) et/ou respectivement de la composition (B).

Selon un mode de réalisation préféré, la composition (A) comprend une ou plusieurs réductones. De manière particulièrement préférée, la ou les réductones sont présentes uniquement dans la composition (A).

De préférence, la quantité totale de réductone(s) dans le mélange des compositions (A) et (B) représente de 0,01 à 1% en poids, de préférence de 0,05 à 0,5% en poids, et mieux encore de 0,1 à 0,2% en poids, par rapport au poids total dudit mélange.

Selon la présente invention, les pourcentages en poids de la ou des réductones sont exprimés par rapport à la forme acide desdites réductones.

De manière connue en soi, le terme « réductone » désigne un composé comprenant une structure ènediol -(HO)C=C(OH)- adjacente à un groupement carbonyle >C=O.

Ainsi, la ou les réductones utilisables dans la présente invention sont de formule générale (II) : 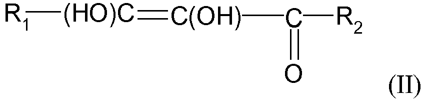 avec R₁ et R₂, identiques ou différents, désignant chacun un groupement contenant au moins un atome de carbone et/ou d'oxygène, R₁ et R₂ pouvant former avec les trois atomes de carbone du composé de formule (II) un cycle de préférence à 5 ou 6 chaînons, dont les atomes constitutifs supplémentaires sont constitués par des atomes de carbone et/ou d'oxygène.

De préférence, R₁ et R₂ forment avec les trois atomes de carbone du composé de formule (II) un cycle à 5 atomes de carbone et/ou d'oxygène.

Le ou les composés de formule (II) peuvent se présenter sous forme acide, ou sous forme de sels, notamment sous forme de sels de métaux alcalins tels que le sodium et le potassium, ou de sels de métaux alcalino-terreux tels que le calcium et le magnésium, ou sous forme d'esters notamment acides gras en C₈ à C₃₀.

De manière particulièrement préférée, le composé de formule (II) est une lactone.

La ou les réductones peuvent être notamment choisies parmi l'acide réductique, l'acide ascorbique, l'acide érythorbique ou isoascorbique, et les sels de ces composés, notamment les sels de sodium ou de potassium, le palmitate d'ascorbyle, et les mélanges de ces composés.

De manière particulièrement préférée, la ou les réductones sont choisies parmi l'acide ascorbique, l'acide érythorbique, et les sels de ces composés, notamment les sels de sodium ou de potassium.

Ainsi que cela a été mentionné, l'une au moins des deux compositions (A) et (B) comprend un ou plusieurs corps gras choisis parmi les hydrocarbures liquides, la quantité totale desdits hydrocarbures liquides dans le mélange des compositions (A) et (B) représentant au moins 20% en poids, par rapport au poids total dudit mélange.

Ainsi, ce ou ces corps gras particuliers peuvent être présents dans la composition (A), dans la composition (B), ou dans les deux compositions à la fois, pourvu que leur quantité totale dans le mélange des compositions (A) et (B) soit au moins égale à 20% en poids, par rapport au poids total du mélange de ces deux compositions.

De préférence, la quantité totale desdits hydrocarbures liquides dans le mélange des compositions (A) et (B) représente au moins 25% en poids, et plus préférentiellement au moins 30% en poids, par rapport au poids total dudit mélange.

La quantité totale desdits hydrocarbures liquides dans le mélange des compositions (A) et (B) est avantageusement inférieure ou égale à 90% en poids, et de préférence inférieure ou égale à 70% en poids, par rapport au poids total dudit mélange.

Selon un mode de réalisation préféré, la composition (A) comprend un ou plusieurs corps gras choisis parmi les hydrocarbures liquides.

Par corps gras, on entend dans la présente invention, un composé organique insoluble dans l'eau à température ordinaire (25°C) et à pression atmosphérique (760 mm de Hg), c'est-à-dire qui présente une solubilité dans l'eau inférieure à 5% en poids et de préférence à 1% en poids, et encore plus préférentiellement inférieure à 0,1% en poids. Les corps gras présentent dans leur structure au moins un enchaînement d'au moins deux groupements siloxanes ou une chaîne hydrocarbonée comportant au moins 6 atomes de carbone. En outre, les corps gras sont généralement solubles dans les solvants organiques dans les mêmes conditions de température et de pression, comme par exemple le chloroforme, l'éthanol, le benzène, l'huile de vaseline ou le décaméthylcyclopentasiloxane.

Selon l'invention, le ou les corps gras sont choisis parmi les hydrocarbures liquides.

Par hydrocarbure liquide, on entend un hydrocarbure composé uniquement d'atomes de carbone et d'hydrogène, liquide à la température ordinaire (25°C) et à pression atmosphérique (760 mm de Hg ; soit 1,013.10⁵ Pa).

Plus particulièrement, les hydrocarbures liquides selon l'invention sont choisis parmi :
- les alcanes inférieurs en C₆-C₁₆ linéaires ou ramifiés, éventuellement cycliques. A titre d'exemple, on peut citer l'hexane, l'undécane, le dodécane, le tridécane, les isoparaffines comme l'isohexadécane, l'isododécane et l'isodécane.
- les hydrocarbures linéaires ou ramifiés, d'origine minérale animale ou synthétique, contenant plus de 16 atomes de carbone, tels que les huiles de paraffine, l'huile de vaseline, les polydécènes, les polyisobutènes hydrogénés tels que le Parléam^{®}, le squalane.

Dans une variante préférée de l'invention, le ou les hydrocarbures liquides sont choisis parmi les huiles de paraffine, l'huile de vaseline, et leurs mélanges.

La composition (A) et/ou la composition (B) selon la présente invention peuvent également comprendre un ou plusieurs autres corps gras additionnels, différents des hydrocarbures liquides ci-avant, et qui ne contiennent pas de fonction acide carboxylique.

Par corps gras ne contenant pas de fonction acide carboxylique, on désigne un corps gras ne contenant pas de groupement -COOH, ni de groupement -COO⁻.

Lesdits corps gras additionnels peuvent notamment être choisis parmi les huiles non siliconées d'origine végétale ou synthétique, autres que les hydrocarbures liquides de l'invention, les alcools gras, les esters d'acide gras et/ou d'alcool gras, les cires non siliconées, les silicones.

Il est rappelé qu'au sens de l'invention, les alcools, esters et acides gras présentent plus particulièrement au moins un groupement hydrocarboné, linéaire ou ramifié, saturé ou insaturé, comprenant 6 à 30 atomes de carbone, éventuellement substitué, en particulier par un ou plusieurs groupements hydroxyle (en particulier 1 à 4). S'ils sont insaturés, ces composés peuvent comprendre une à trois double-liaisons carbone-carbone, conjuguées ou non.

Comme huiles d'origine végétale ou synthétique, utilisables dans l'agent selon l'invention, on peut citer par exemple :
- les huiles triglycérides d'origine végétale ou synthétique, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations Miglyol^{®} 810, 812 et 818 par la société Dynamit Nobel, l'huile de jojoba, l'huile de beurre de karité.
- les huiles fluorées comme le perfluorométhylcyclopentane et le perfluoro-1,3 diméthylcyclohexane, vendus sous les dénominations de "FLUTEC^{®} PC1" et "FLUTEC^{®} PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050^{®}" et "PF 5060^{®}" par la Société 3M, ou encore le bromoperfluorooctyle vendu sous la dénomination "FORALKYL^{®}" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052^{®}" par la Société 3M.

On peut aussi utiliser la vaseline.

Les alcools gras convenant à la mise en oeuvre de l'invention sont plus particulièrement choisis parmi les alcools saturés ou insaturés, linéaires ou ramifiés, comportant de 8 à 30 atomes de carbone. On peut citer par exemple l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

En ce qui concerne les esters d'acide gras et/ou d'alcool gras, avantageusement différents des triglycérides mentionnés ci-dessus, on peut citer notamment les esters de mono ou polyacides aliphatiques saturés ou insaturés, linéaires ou ramifiés en C₁-C₂₆ et de mono ou polyalcools aliphatiques saturés ou insaturés, linéaires ou ramifiés en C₁-C₂₆, le nombre total de carbone des esters étant supérieur ou égal à 10.

Parmi les monoesters, on peut citer le béhénate de dihydroabiétyle ; le béhénate d'octyldodécyle ; le béhénate d'isocétyle; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-hexyle, le palmitate de 2-octyldécyle, les myristates d'alkyles tels que le myristate d'isopropyle, de butyle, de cétyle, de 2-octyldodécyle, de mirystyle, de stéaryle le stéarate d'hexyle, le stéarate de butyle, le stéarate d'isobutyle ; le maléate de dioctyle, le laurate d'hexyle, le laurate de 2-hexyldécyle.

Toujours dans le cadre de cette variante, on peut également utiliser les esters d'acides di ou tricarboxyliques en C₄-C₂₂ et d'alcools en C₁-C₂₂ et les esters d'acides mono di ou tricarboxyliques et d'alcools di, tri, tétra ou pentahydroxy en C₂-C₂₆.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-hexyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le maléate de dioctyle, le laurate d'hexyle, le laurate de 2-hexyldécyle et l'isononanate d'isononyle, l'octanoate de cétyle.

L'agent selon l'invention peut également comprendre, à titre d'ester gras, des esters et di-esters de sucres d'acides gras en C₆-C₃₀, de préférence en C₁₂-C₂₂. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C₆-C₃₀, de préférence en C₁₂-C₂₂, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou di- oléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

La cire ou les cires (non siliconées) sont choisies notamment parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina); d'autres cires ou matières premières cireuses utilisables selon l'invention sont notamment les cires marines telles que celle vendue par la Société SOPHIM sous la référence M82, les cires de polyéthylène ou de polyoléfines en général.

Les silicones utilisables dans l'agent selon la présente invention, sont des silicones volatiles ou non volatiles, cycliques, linéaires ou ramifiées, modifiées ou non par des groupements organiques, ayant une viscosité de 5.10⁻⁶ à 2,5m²/s à 25°C et de préférence 1.10⁻⁵ à 1m²/s.

Les silicones utilisables conformément à l'invention peuvent se présenter sous forme d'huiles, de cires, de résines ou de gommes.

De préférence, la silicone est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
(i) les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE^{®} 7207 par UNION CARBIDE ou SILBIONE^{®} 70045 V2 par RHODIA, le décaméthylcyclopentasiloxane commercialisé sous le nom de VOLATILE SILICONE^{®} 7158 par UNION CARBIDE, et SILBIONE® 70045 V5 par RHODIA, ainsi que leurs mélanges.

On peut également citer les cyclocopolymères du type diméthylsiloxanes/ méthylalkylsiloxane, tel que la SILICONE VOLATILE^{®} FZ 3109 commercialisée par la société UNION CARBIDE, de formule :

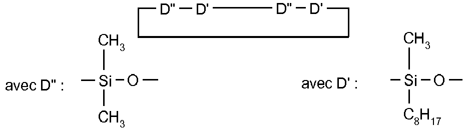

On peut également citer les mélanges de polydialkylsiloxanes cycliques avec des composés organiques dérivés du silicium, tels que le mélange d'octaméthylcyclotétrasiloxane et de tétratriméthylsilylpentaérythritol 1 (50/50) et le mélange d'octaméthylcyclotétrasiloxane et d'oxy-1 l'-(hexa-2,2,2',2',3,3'-triméthylsilyloxy) bis-néopentane ;
(ii)les polydialkylsiloxanes volatiles linéaires ayant 2 à 9 atomes de silicium et présentant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm²/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl (C₁-C₂₀) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
R₂SiO_{2/2}, R₃SiO_{1/2}, RSiO_{3/2} et SiO_{4/2}
dans lesquelles R représente un alkyl possédant 1 à 16 atomes de carbone. Parmi ces produits, ceux particulièrement préférés sont ceux dans lesquels R désigne un groupe alkyle inférieur en C₁-C₄, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkyl-arylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl (C₁₂)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

De préférence, le ou les corps gras additionnels sont liquides à la température de 25°C et à la pression atmosphérique.

Le ou les corps gras additionnels sont de préférence choisis parmi les alcools gras, les esters d'acide gras, les esters d'alcool gras, les huiles non siliconées végétales ou synthétiques différentes des hydrocarbures liquides de l'invention, les silicones, et leurs mélanges.

De manière plus préférée, le ou les corps gras additionnel sont choisis parmi les esters d'acides gras et/ou d'alcools gras liquides, les alcools gras liquides ou leurs mélanges.

La quantité totale desdits corps gras additionnels dans le mélange des compositions (A) et (B) peut représenter de 0,1 à 40 % en poids, plus préférentiellement de 0,5 à 25 % en poids, et mieux de 1 à 20 % en poids, par rapport au poids total dudit mélange.

Selon un mode de réalisation préféré, la composition (A) comprend un ou plusieurs corps gras additionnels tels que décrits ci-avant, en une quantité préférentielle allant de 1 à 20% en poids, par rapport au poids total de la composition (A).

Les compositions (A) et/ou (B) selon la présente invention peuvent comprendre en outre un ou plusieurs tensioactifs.

Dans ce cas, le ou les tensioactifs sont de préférence choisis parmi les tensioactifs non ioniques, ou parmi les tensioactifs anioniques.

Les tensioactifs anioniques sont plus spécialement choisis parmi les sels (en particulier sels de métaux alcalins, notamment de sodium, sels d'ammonium, sels d'amines tels que les sels d'aminoalcools ou sels de métaux alcalino-terreux comme le magnésium) des composés suivants :
- les alkylsulfates, les alkyléthersulfates, alkylamidoéthersulfates, alkylaryl-polyéthersulfates, monoglycérides sulfates ;
- les alkylsulfonates, alkylamidesulfonates, alkylarylsulfonates, α-oléfine-sulfonates, paraffine-sulfonates ;
- les alkylphosphates, les alkylétherphosphates;
- les alkylsulfosuccinates, les alkyléthersulfosuccinates, les alkylamide-sulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

Il est à noter que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.

Les tensioactifs non ioniques sont plus particulièrement choisis parmi les tensioactifs non ioniques mono ou poly- oxyalkylénés, mono- ou poly- glycérolés. Les motifs oxyalkylénés sont plus particulièrement des motifs oxyéthylénés, oxypropylénés, ou leur combinaison, de préférence oxyéthylénés.

A titre d'exemples de tensioactifs non ioniques oxyalkylénés, on peut citer :
- les alkyl(C₈-C₂₄)phénols oxyalkylénés,
- les alcools en C₈-C₃₀, saturés ou non, linéaires ou ramifiés, oxyalkylénés,
- les amides, en C₈-C₃₀, saturés ou non, linéaires ou ramifiés, oxyalkylénés,
- les esters d'acides en C₈-C₃₀, saturés ou non, linéaires ou ramifiés, et de polyéthylèneglycols,
- les esters d'acides en C₈-C₃₀, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène, entre autres,
- et leurs mélanges.

Ces tensioactifs présentent un nombre de moles d'oxyde d'éthylène et/ou de propylène compris entre 1 et 100, de préférence entre 2 et 50. De manière avantageuse, les tensioactifs non ioniques ne comprennent pas de motifs oxypropylénés.

Conformément à un mode de réalisation préféré de l'invention, les tensioactifs non ioniques oxyalkylénés sont choisis parmi les alcools en C₈-C₃₀, oxyéthylénés, les esters d'acides en C₈-C₃₀, saturés ou non, linéaires ou ramifiés et de sorbitol polyoxythylénés.

A titre d'exemple de tensioactifs non ioniques mono- ou poly-glycérolés, on utilise de préférence les alcools en C₈-C₄₀, mono- ou poly- glycérolés.

En particulier, les alcools en C₈-C₄₀ mono- ou poly- glycérolés correspondent à la formule suivante :

RO-[CH₂-CH(CH₂OH)-O]ₘ-H

dans laquelle R représente un radical alkyle ou alcényle, linéaire ou ramifié, en C₈-C₄₀, de préférence en C₈-C₃₀, et m représente un nombre allant de 1 à 30 et de préférence de 1 à 10.

A titre d'exemple de composés convenables dans le cadre de l'invention, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en C₈/C₁₀ à une mole de glycérol, l'alcool en C₁₀/C₁₂ à 1 mole de glycérol et l'alcool en C₁₂ à 1,5 mole de glycérol.

Selon la présente invention, on préfère employer un ou plusieurs tensioactifs non ioniques.

Selon un mode de réalisation préféré, la composition (A) comprend un ou plusieurs tensioactifs.

Le ou les tensioactifs peuvent être présents dans des proportions allant de 0,1 à 50% en poids, de préférence de 0,5 à 30 % en poids, par rapport au poids total de chaque composition dans laquelle ils sont contenus.

Les compositions (A) et/ou (B) selon la présente invention peuvent également comprendre un ou plusieurs agents épaississants minéraux choisis parmi les argiles organophiles, les silices pyrogénées, ou leurs mélanges.

L'argile organophile peut être choisie parmi la montmorrilonite, la bentonite, l'hectorite, l'attapulgite, la sépiolite, et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxydes amines, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leur surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de la silice par réaction chimique en vue de diminuer le nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

Les agents épaississants minéraux préférés sont choisis parmi les hectorites, les bentonites organomodifiées, et les silices pyrogénées éventuellement modifiées.

Lorsqu'il est présent, l'agent épaississant minéral représente de 1 à 30 % en poids par rapport au poids de la composition dans laquelle il est présent.

Les compositions (A) et/ou (B) selon la présente invention peuvent également comprendre un ou plusieurs agents épaississants organiques.

Ces agents épaississants peuvent être choisis parmi les amides d'acides gras (diéthanol- ou monoéthanol-amide de coprah, monoéthanolamide d'acide alkyl éther carboxylique oxyéthyléné), les épaississants polymériques tels que les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique et les polymères associatifs (polymères comprenant des zones hydrophiles, et des zones hydrophobes à chaîne grasse (alkyle, alcényle comprenant au moins 10 atomes de carbone) capables, dans un milieu aqueux, de s'associer réversiblement entre eux ou avec d'autres molécules).

Selon un mode de réalisation particulier, l'épaississant organique est choisi parmi les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique, et de préférence parmi les épaississants cellulosiques avec en particulier l'hydroxyéthycellulose.

La teneur en agent(s) épaississant(s) organique(s), s'ils sont présents, varie habituellement de 0,01 % à 20 % en poids, de préférence de 0,1 à 5 % en poids, par rapport au poids de chaque composition dans laquelle ils sont présents.

Avantageusement, la composition (A) se présente sous la forme d'un gel ou d'une crème.

Avantageusement, la composition (B) se présente sous la forme d'une solution, d'une émulsion ou d'un gel.

Les colorants d'oxydation utilisables dans la présente invention sont en général choisis parmi les bases d'oxydation, éventuellement combinée(s) à un ou plusieurs coupleurs.

Les bases d'oxydation peuvent être notamment choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[l,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(3-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

De préférence, on utilisera un 4,5-diaminopyrazole et encore plus préférentiellement le 4,5-diamino-1-(β-hydroxyéthyl)-pyrazole et/ou l'un des ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1-yl)-1,2-diéthyl-1,2-dihydro-pyrazol-3-one, 2,3-diamino-6-hydroxy-6,7-dihydro-1H,5H-pyrazolo[1,2-a]pyrazol-1-one.

On préférera utiliser la 2,3-diamino-6,7-dihydro-1H,5H-pyrazolo[1,2-a]pyrazol-1-one et/ou un de ses sels.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels.

Les coupleurs utilisables dans la présente invention peuvent être choisis parmi ceux conventionnellement utilisés pour la teinture des fibres kératiniques.

Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(ß-hydroxyéthyloxy) benzène, le 2-amino 4-(ß-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-ß-hydroxyéthylamino-3,4-méthylènedioxybenzène, l'α-naphtol, le 2-méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(ß-hydroxyéthyl)amino-3,4-méthylène dioxybenzène, le 2,6-bis-(ß-hydroxyéthylamino)toluène, la 6-hydroxy indoline, la 2,6-dihydroxy 4-méthyl pyridine, la 1-H-3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation peuvent représenter chacune avantageusement de 0,0001 à 10 % en poids par rapport au poids total de la composition (A), et de préférence de 0,005 à 5 % en poids par rapport au poids total de cette composition.

Le ou les coupleurs, s'ils sont présents, peuvent représenter chacun avantageusement de 0,0001 à 10 % en poids par rapport au poids total de la composition (A), et de préférence de 0,005 à 5 % en poids par rapport au poids total de cette composition.

Les colorants directs susceptibles d'être employés dans la composition (A) sont plus particulièrement choisis parmi les espèces ioniques ou non ioniques, de préférence cationiques ou non ioniques.

A titre d'exemples de colorants directs convenables, on peut citer les colorants directs azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

Plus particulièrement, les colorants azoïques comprennent une fonction -N=N- dont les deux atomes d'azote ne sont pas simultanément engagés dans un cycle. Il n'est toutefois pas exclu que l'un des deux atomes d'azote de l'enchaînement -N=N- soit engagé dans un cycle.

Les colorants de la famille des méthines sont plus particulièrement des composés comprenant au moins un enchaînement choisi parmi >C=C< et -N=C< dont les deux atomes ne sont pas simultanément engagés dans un cycle. Il est toutefois précisé que l'un des atomes d'azote ou de carbone des enchaînements peut être engagé dans un cycle. Plus particulièrement, les colorants de cette famille sont issus de composés de type méthine, azométhine, mono- et di-arylméthane, indoamines (ou diphénylamines), indophénols, indoanilines, carbocyanines, azacabocyanines et leurs isomères, diazacarbocyanines et leurs isomères, tétraazacarbocyanines, hémicyanines.

Concernant les colorants de la famille des carbonyles, on peut citer par exemple les colorants choisis parmi les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.

Concernant les colorants de la famille des azines cycliques, on peut citer notamment les azine, xanthène, thioxanthène, fluorindine, acridine, (di)oxazine, (di)thiazine, pyronine.

Les colorants nitrés (hétéro)aromatiques sont plus particulièrement des colorants directs nitrés benzéniques ou nitrés pyridiniques.

Concernant les colorants de type porphyrines ou phtalocyanines, on peut mettre en oeuvre des composés cationiques ou non, comprenant éventuellement un ou plusieurs métaux ou ions métalliques, comme par exemple des métaux alcalins et alcalino-terreux, le zinc et le silicium.

A titre d'exemple de colorants directs particulièrement convenables, on peut citer les colorants nitrés de la série benzénique ; les colorants directs azoïques ; azométhiniques ; méthiniques ; les azacarbocyanines comme les tétraazacarbocyanines (tétraazapentaméthines) ; les colorants directs quinoniques et en particulier anthraquinoniques, naphtoquinoniques ou benzoquinoniques ; les colorants directs aziniques ; xanthéniques ; triarylméthaniques ; indoaminiques ; indigoïdes ; phtalocyanines, porphyrines et les colorants directs naturels, seuls ou en mélanges.

Ces colorants peuvent être des colorants monochromophoriques (c'est-à-dire ne comprenant qu'un seul colorant) ou polychromophoriques, de préférence di- ou tri- chromophoriques ; les chromophores pouvant être identiques ou non, de la même famille chimique ou non. A noter que qu'un colorant polychromophorique comprend plusieurs radicaux issus chacun d'une molécule absorbant dans le domaine visible entre 400 et 800 nm. De plus cette absorbance du colorant ne nécessite ni oxydation préalable de celui-ci, ni association avec d'autre(s) espèce(s) chimique(s).

Dans le cas de colorants polychromophoriques, les chromophores sont reliés entre eux au moyen d'au moins un bras de liaison qui peut être cationique ou non.

De préférence, le bras de liaison est une chaîne alkyle en C₁-C₂₀, linéaire, ramifiée ou cyclique, éventuellement interrompue par au moins un hétéroatome (tel que l'azote, l'oxygène) et/ou par au moins un groupe en comprenant (CO, SO₂), éventuellement interrompue par au moins un hétérocycle condensé ou non avec un noyau phényle et comprenant au moins un atome d'azote quaternisé engagé dans ledit cycle et éventuellement au moins un autre hétéroatome (tel que l'oxygène, l'azote ou le soufre), éventuellement interrompue par au moins un groupement phényle ou naphtyle substitué ou non, éventuellement au moins un groupement ammonium quaternaire substitué par deux groupements alkyle en C₁-C₁₅ éventuellement substitués ; le bras de liaison ne comprenant pas de groupement nitro, nitroso ou peroxo.

Si les hétérocycles ou noyaux aromatiques sont substitués, ils le sont par exemple par un ou plusieurs radicaux alkyle en C₁-C₈ éventuellement substitués par un groupement hydroxy, alcoxy en C₁-C₂, hydroxyalcoxy en C₂-C₄, acétylamino, amino substitué par un ou deux radicaux alkyle en C₁-C₄, éventuellement porteurs d'au moins un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle ; un radical alcoxy en C₁-C₂ ; un radical hydroxyalcoxy en C₂-C₄ ; un radical amino ; un radical amino substitué par un ou deux radicaux alkyle, identiques ou différents, en C₁-C₄ éventuellement porteurs d'au moins un groupement hydroxyle.

Parmi les colorants directs benzéniques utilisables selon l'invention, on peut citer de manière non limitative les composés suivants :
- 1,4-diamino-2-nitrobenzène,
- 1-amino-2 nitro,-4-β-hydroxyéthylaminobenzène
- 1-amino-2 nitro-4-bis(β-hydroxyéthyl)-aminobenzène
- 1,4-bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(ß-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-diamino-4-nitrobenzène
- 1-amino-2-β-hydroxyéthylamino-5-nitrobenzène
- 1,2-bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, azométhines, méthines ou tétraazapentaméthines utilisables selon l'invention on peut citer les colorants cationiques décrits dans les demandes de brevets WO 95/15144, WO 95/01772 et EP 714954 ; FR 2189006, FR 2285851, FR-2140205, EP 1378544, EP 1674073.

Ainsi, on peut tout notamment citer les colorants suivants de formules (I) à (IV) ci-après, et de préférence les composés de formules (I) et (III) suivantes : 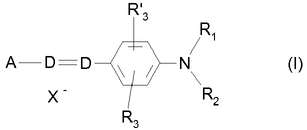 dans laquelle :
D représente un atome d'azote ou le groupement -CH,
R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₃ et R'₃, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyloxy,
X ⁻ représente un anion de préférence choisi parmi le chlorure, le méthyl sulfate et l'acétate,
A représente un groupement choisi par les structures A1 à A18, de préférence A1, A4, A7, A13 et A18 suivantes : 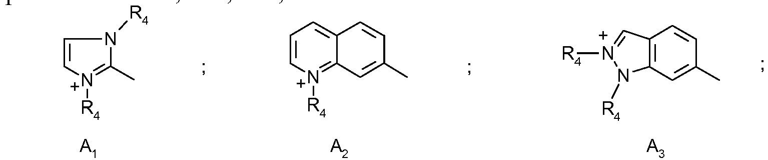 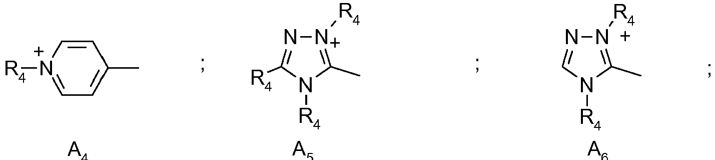 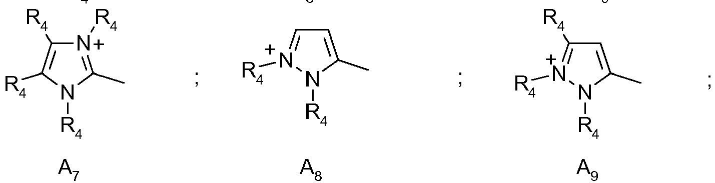 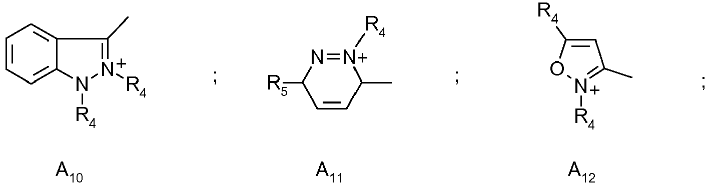 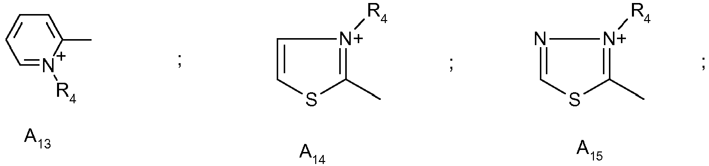 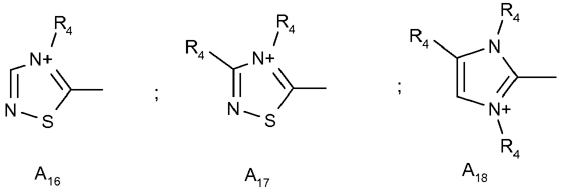
dans lesquelles R₄ représente un radical alkyle en C₁-C₄ pouvant être substitué par un radical hydroxyle et R₅ représente un radical alcoxy en C₁-C₄ ; 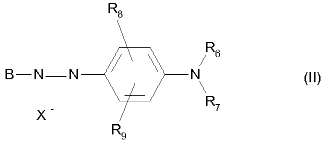 dans laquelle :
   R₆ représente un atome d'hydrogène ou un radical alkyle en C₁-C₄,
   R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
   R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical - CN,
   X⁻ représente un anion de préférence choisi parmi le chlorure, le méthyl sulfate et l'acétate,
   B représente un groupement choisi par les structures B1 à B6 suivantes : 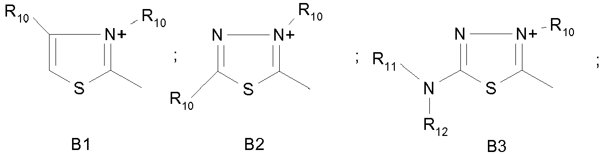 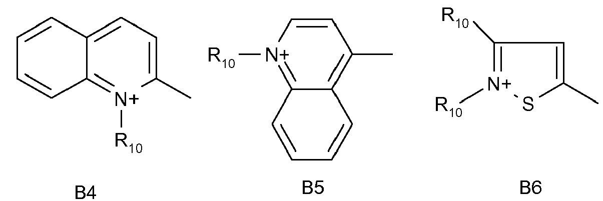
   dans lesquelles R₁₀ représente un radical alkyle en C₁-C₄, R₁₁ et R₁₂, identiques ou différents, représentent un atome d'hydrogène ou un radical alkyle en C₁-C₄ ; 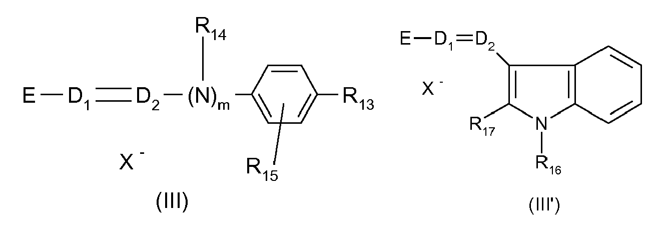 dans lesquelles : R₁₃ représente un atome d'hydrogène, un radical alcoxy en C₁-C_{4,} un atome d'halogène tel que le brome, le chlore, l'iode ou le fluor,
      R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
      R₁₅ représente un atome d'hydrogène ou d'halogène tel que le brome, le chlore, l'iode ou le fluor,
      R₁₆ et R₁₇, identiques ou différents, représentent un atome d'hydrogène ou un radical alkyle en C₁-C₄,
      D₁ et D₂, identiques ou différents, représentent un atome d'azote ou le groupement -CH,
      m = 0 ou 1, de préférence 1,
      étant entendu que lorsque R₁₃ représente un groupement amino non substitué, alors D₁ et D₂ représentent simultanément un groupement -CH et m = 0,
      X ⁻ représente un anion de préférence choisi parmi le chlorure, le méthyl sulfate et l'acétate,
      E représente un groupement choisi par les structures E1 à E8, plus particulièrement E1, E2 et E7, suivantes : 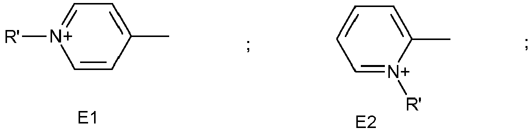 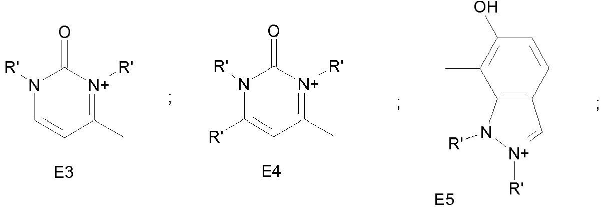 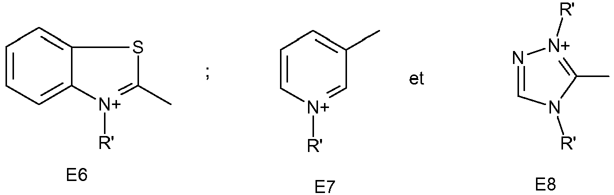
      dans lesquelles R' représente un radical alkyle en C₁-C₄ ;
      lorsque m = 0 et que D₁ représente un atome d'azote, alors E peut également désigner un groupement de structure E9 suivante : 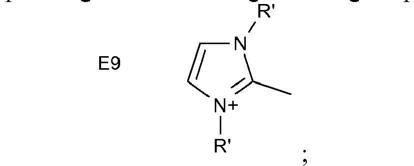
         dans laquelle R' représente un radical alkyle en C₁-C₄.

G-N=N-J (IV)

dans laquelle :
le symbole G représente un groupement choisi parmi les structures G₁ à G₃ suivantes : 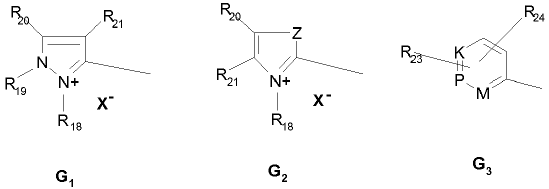 structures G₁ à G₃ dans lesquelles,
   R₁₈ désigne un radical alkyle en C₁-C₄, un radical phényle pouvant être substitué par un radical alkyle en C₁-C₄ ou un atome d'halogène choisi parmi le chlore, le brome, l'iode et le fluor ;
   R₁₉ désigne un radical alkyle en C₁-C₄ ou un radical phényle;
   R₂₀ et R₂₁, identiques ou différents, représentent un radical alkyle en C₁-C₄, un radical phényle, ou forment ensemble dans G₁ un cycle benzénique substitué par un ou plusieurs radicaux alkyle en C₁-C₄, alcoxy en C₁-C₄, ou NO₂, ou forment ensemble dans G₂ un cycle benzénique éventuellement substitué par un ou plusieurs radicaux alkyle en C₁-C₄, alcoxy en C₁-C₄, ou NO₂ ;
   R₂₀ peut désigner en outre un atome d'hydrogène ;
   Z désigne un atome d'oxygène, de soufre ou un groupement -NR₁₉ ;
   M représente un groupement -CH, -CR (R désignant alkyle en C₁-C₄),
   ou -NR₂₂(X⁻)ᵣ;
   K représente un groupement -CH, -CR (R désignant alkyle en C₁-C₄),
   ou -NR₂₂(X⁻)ᵣ;
   P représente un groupement -CH, -CR (R désignant alkyle en C₁-C₄),
   ou -NR₂₂(X⁻)ᵣ; r désigne zéro ou 1;
   R₂₂ représente un atome O⁻, un radical alcoxy en C₁-C₄, ou un radical alkyle en C₁-C₄;
   R₂₃ et R₂₄, identiques ou différents, représentent un atome d'hydrogène ou d'halogène choisi parmi le chlore, le brome, l'iode et le fluor, un radical alkyle en C₁-C₄, alcoxy en C₁-C₄ , un radical -NO₂;
   X⁻ représente un anion de préférence choisi parmi le chlorure, l'iodure, le méthyl sulfate, l'éthyl sulfate, l'acétate et le perchlorate;
   sous réserve que,
   si R₂₂ désigne O⁻, alors r désigne zéro;
   si K ou P ou M désignent -N-alkyle C₁-C₄ X⁻, alors R₂₃ ou R₂₄ est de préférence différent d'un atome d'hydrogène;
   si K désigne -NR₂₂(X⁻)ᵣ, alors M= P= -CH, -CR;
   si M désigne -NR₂₂(X⁻)ᵣ, alors K= P= -CH, -CR;
   si P désigne -NR₂₂(X⁻)ᵣ, alors K= M et désignent -CH ou -CR;
   si Z désigne un atome de soufre avec R₂₁ désignant alkyle en C₁-C₄, alors R₂₀ est différent d'un atome d'hydrogène;
   si Z désigne -NR₂₂ avec R₁₉ désignant alkyle en C₁-C₄, alors au moins l'un des radicaux R₁₈, R₂₀ ou R₂₁ du groupement de structure G₂ est différent d'un radical alkyle en C₁-C₄;
le symbole J représente :
   - (a) un groupement de structure J₁ suivante : 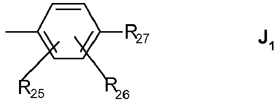 structure J₁ dans laquelle,
      R₂₅ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₂₈, -NR₂₉R₃₀, -NHCOalkyle en C₁-C₄, ou forme avec R₂₆ un cycle à 5 ou 6 chaînons contenant ou non un ou plusieurs hétéroatomes choisis parmi l'azote, l'oxygène ou le soufre;
      R₂₆ représente un atome d'hydrogène, un atome d'halogène choisi parmi le chlore, le brome, l'iode et le fluor, un radical alkyle en C₁-C₄, alcoxy en C₁-C₄,
      ou forme avec R₂₇ ou R₂₈ un cycle à 5 ou 6 chaînons contenant ou non un ou plusieurs hétéroatomes choisis parmi l'azote, l'oxygène ou le soufre ;
      R₂₇ représente un atome d'hydrogène, un radical -OH, un radical -NHR₂₈, un radical -NR₂₉R₃₀ ;
      R₂₈ représente un atome d'hydrogène, un radical alkyle en C₁-C₄, un radical monohydroxyalkyle en C₁-C₄, polyhydroxyalkyle en C₂-C₄, un radical phényle;
      R₂₉ et R₃₀, identiques ou différents, représentent un radical alkyle en C₁-C₄, un radical monohydroxyalkyle en C₁-C₄, polyhydroxyalkyle en C₂-C₄ ;
   - (b) un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle,
      et notamment un groupement de structure J₂ suivante : 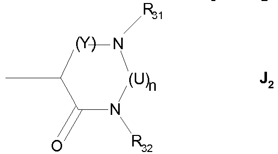 structure J₂ dans laquelle,
      R₃₁ et R₃₂, identiques ou différents, représentent un atome d'hydrogène, un radical alkyle en C₁-C₄, un radical phényle;
      Y désigne le radical -CO- ou le radical 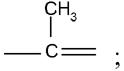
      n = 0 ou 1, avec, lorsque n désigne 1, U désigne le radical - CO- .

Dans les structures des colorants (I) à (IV) définies ci-dessus, le groupement alkyle ou alcoxy en C₁-C₄ désigne de préférence méthyle, éthyle, butyle, méthoxy, éthoxy.

Parmi les colorants de formules (I) et (III), on préfère les composés suivants :

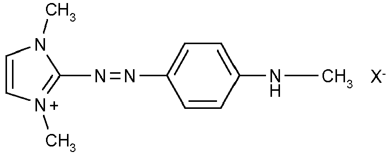

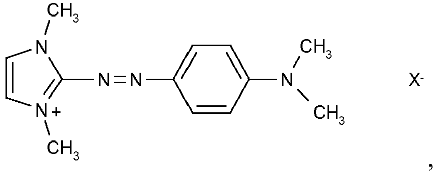

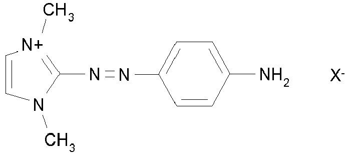

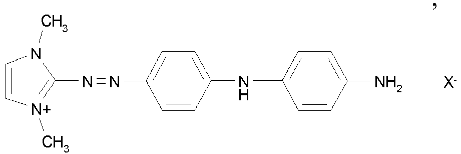

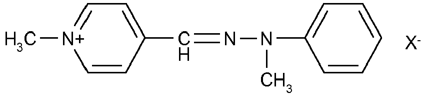

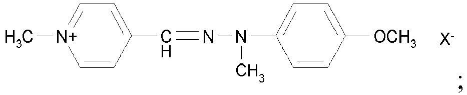

On peut également citer parmi les colorants directs azoïques les colorants suivants, décrits dans le COLOUR INDEX INTERNATIONAL 3e édition :
- Disperse Red 17
- Basic Red 22
- Basic Red 76
- Basic Yellow 57
- Basic Brown 16
- Basic Brown 17
- Disperse Black 9.

On peut aussi citer le 1-(4'-aminodiphénylazo)-2-méthyl-4bis-(β-hydroxyéthyl) aminobenzène.

Parmi les colorants directs quinoniques on peut citer les colorants suivants :
- Disperse Red 15
- Solvent Violet 13
- Disperse Violet 1
- Disperse Violet 4
- Disperse Blue 1
- Disperse Violet 8
- Disperse Blue 3
- Disperse Red 11
- Disperse Blue 7
- Basic Blue 22
- Disperse Violet 15
- Basic Blue 99
ainsi que les composés suivants :
- 1-N-méthylmorpholiniumpropylamino-4-hydroxy anthraquinone
- 1-Aminopropylamino-4-méthylaminoanthraquinone
- 1-Aminopropylamino anthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :
- Basic Blue 17
- Basic Red 2.

Parmi les colorants triarylméthaniques utilisables selon l'invention, on peut citer les composés suivants :
- Basic Green 1
- Basic Violet 3
- Basic Violet 14
- Basic Blue 7
- Basic Blue 26

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants figurant dans le tableau ci-dessous :

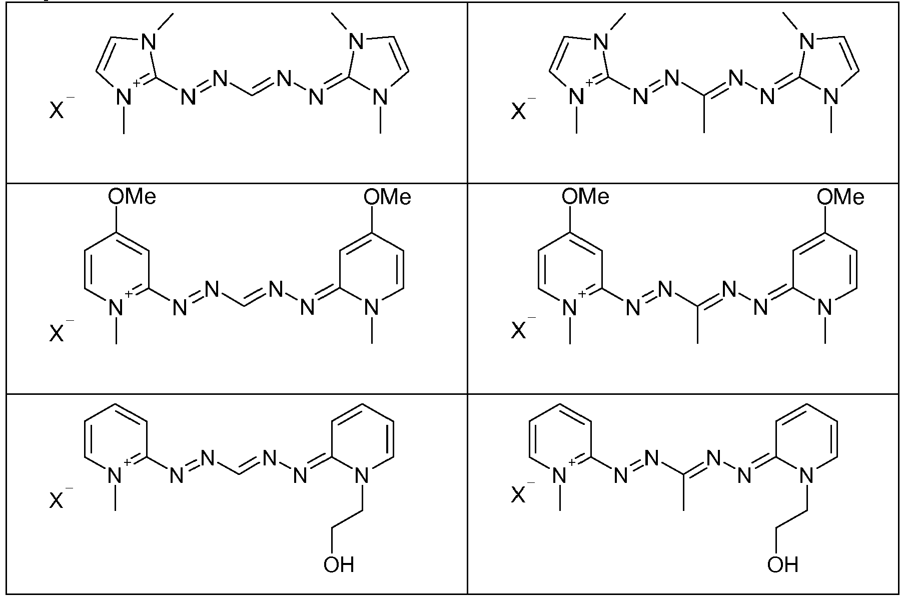

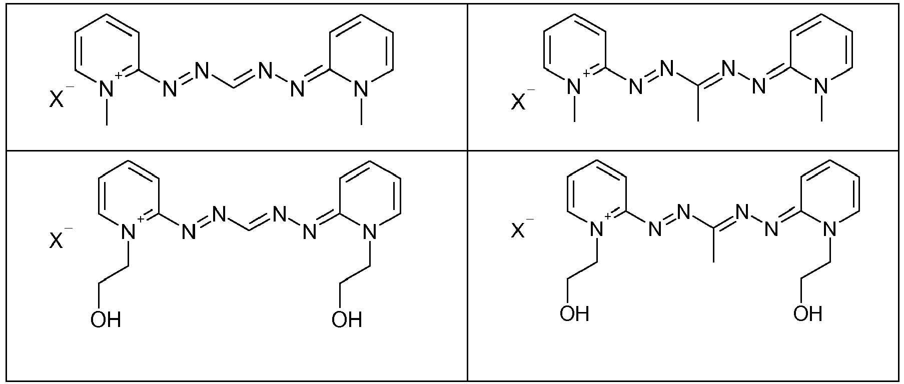

X représente un anion de préférence choisi parmi le chlorure, l'iodure, le méthyl sulfate, l'éthyl sulfate, l'acétate et le perchlorate.

Parmi les colorants polychromophoriques, on peut citer plus particulièrement les colorants di- ou tri- chromophoriques azoïques et/ou azométhiniques (hydrazoniques), symétriques ou non, comprenant d'une part au moins un hétérocycle aromatique comprenant 5 ou 6 chaînons, éventuellement condensé, comprenant au moins un atome d'azote quaternisé engagé dans ledit hétérocycle et éventuellement au moins un autre hétéroatome (tel que l'azote, le soufre, l'oxygène), et d'autre part, au moins un groupement phényle ou naphtyle, éventuellement substitué, éventuellement porteur d'au moins un groupement OR avec R représentant un atome d'hydrogène, un radical alkyle éventuellement substitué en C₁-C₆, un noyau phényle éventuellement substitué, ou d'au moins un groupement N(R')₂ avec R' identiques ou non, représentant un atome d'hydrogène, un radical alkyle éventuellement substitué en C₁-C₆, un noyau phényle éventuellement substitué ; les radicaux R' pouvant former avec l'atome d'azote auquel ils sont liés, un hétérocycle saturé à 5 ou 6 chaînons, ou bien encore l'un et/ou les deux radicaux R' peuvent former chacun avec l'atome de carbone du cycle aromatique placé en ortho de l'atome d'azote, un hétérocycle saturé à 5 ou 6 chaînons.

A titre d'hétérocycle cationique aromatique, on peut citer de préférence, les cycles à 5 ou 6 chaînons comprenant 1 à 3 atomes d'azote, de préférence 1 ou 2 atomes d'azote, l'un étant quaternisé ; ledit hétérocycle étant par ailleurs éventuellement condensé à un noyau benzénique. Il est à noter de même que l'hétérocycle peut éventuellement comprendre un autre hétéroatome différent de l'azote, comme le soufre ou l'oxygène.

Si les hétérocycles ou groupements phényle ou napthyle sont substitués, ils le sont par exemple par un ou plusieurs radicaux alkyle en C₁-C₈ éventuellement substitués par un groupement hydroxy, alcoxy en C₁-C₂, hydroxyalcoxy en C₂-C₄, acétylamino, amino substitué par un ou deux radicaux alkyle en C₁-C₄, éventuellement porteurs d'au moins un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle ; un radical alcoxy en C₁-C₂ ; un radical hydroxyalcoxy en C₂-C₄ ; un radical amino ; un radical amino substitué par un ou deux radicaux alkyle, identiques ou différents, en C₁-C₄ éventuellement porteurs d'au moins un groupement hydroxyle.

Ces polychromophores sont reliés entre eux au moyen d'au moins un bras de liaison comprenant éventuellement au moins un atome d'azote quaternisé engagé ou non dans un hétérocycle saturé ou non, éventuellement aromatique.

De préférence, le bras de liaison est une chaîne alkyle en C₁-C₂₀, linéaire, ramifiée ou cyclique, éventuellement interrompue par au moins un hétéroatome (tel que l'azote, l'oxygène) et/ou par au moins un groupe en comprenant (CO, SO₂), éventuellement interrompue par au moins un hétérocycle condensé ou non avec un noyau phényle et comprenant au moins un atome d'azote quaternisé engagé dans ledit cycle et éventuellement au moins un autre hétéroatome (tel que l'oxygène, l'azote ou le soufre), éventuellement interrompue par au moins un groupement phényle ou naphtyle substitué ou non, éventuellement au moins un groupement ammonium quaternaire substitué par deux groupements alkyle en C₁-C₁₅ éventuellement substitués ; le bras de liaison ne comprenant pas de groupement nitro, nitroso ou peroxo.

La liaison entre le bras de liaison et chaque chromophore se fait en général au moyen d'un hétéroatome substituant le noyau phényle ou napthyle ou au moyen de l'atome d'azote quaternisé de l'hétérocycle cationique.

Le colorant peut comprendre des chromophores identiques ou non.

A titre d'exemples de tels colorants, on pourra notamment se reporter aux demandes de brevets EP 1637566, EP 1619221, EP 1634926, EP 1619220, EP 1672033, EP 1671954, EP 1671955, EP 1679312, EP 1671951, EP167952, EP167971, WO 06/063866, WO 06/063867, WO 06/063868, WO 06/063869, EP 1408919, EP 1377264, EP 1377262, EP 1377261, EP 1377263, EP 1399425, EP 1399117, EP 1416909, EP 1399116, EP 1671560.

On peut aussi également mettre en oeuvre des colorants directs cationiques cités dans les demandes EP 1006153, qui décrit des colorants comprenant deux chromophores de type anthraquinones reliés au moyen d'un bras de liaison cationique ; EP 1433472, EP 1433474, EP 1433471 et EP 1433473 qui décrivent des colorants dichromophoriques identiques ou non, reliés par un bras de liaison cationique ou non, ainsi que EP 6291333 qui décrit notamment des colorants comprenant trois chromophores, l'un d'eux étant un chromophore anthraquinone auquel sont reliés deux chromophores de type azoïque ou diazacarbocyanine ou l'un de ses isomères.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Lorsqu'ils sont présents, le ou les colorants directs représentent avantageusement de 0,0001 à 10% en poids du poids total de la composition (A), et de préférence de 0,005 à 5% en poids.

Dans ce mode de réalisation, la composition (A) peut avantageusement comprendre un ou plusieurs adjuvants solides ou pâteux, et de préférence pulvérulents. Les adjuvants peuvent alors être choisis parmi les argiles, les sels différents des sels d'ammonium, les tensioactifs anioniques, non ioniques, cationiques ou zwittérioniques, les épaississants naturels ou de synthèse, l'amidon éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono- ou disaccharides comme le glucose, le saccharose, le sorbitol ou le fructose, l'oxyde de zinc, de zirconium, les silicabades, le talc, les borosilicates notamment de calcium, le polyéthylène, le polytétrafluoroéthylène (PTFE), la cellulose et ses dérivés, les composés superabsorbants, les carbonates de magnésium ou de calcium, le polyacrylamide, l'hydroxyapatite poreux, la sciure de bois, la poudre de fucus, la polyvinylpyrrolidone réticulée, l'alginate de calcium, le charbon actif, les particules de poly(chlorure de vinylidène/acrylonitrile), notamment celles commercialisées sous la dénomination générale d'« Expancel^{®} » par la société AKZO NOBEL sous les références particulières « Expancel^{®} WE» ou « DE » Expancels, et leurs mélanges.

D'une manière générale, les compositions (A) et (B) sont formulées dans un milieu cosmétiquement acceptable, qui comprend généralement de l'eau et/ou un ou plusieurs solvants organiques.

A titre de solvant organique, on peut par exemple citer les alcanols, linéaires ou ramifiés, en C₂-C₄, tels que l'éthanol et l'isopropanol ; le glycérol ; les polyols et éthers de polyols comme le 2-butoxyéthanol, le propylèneglycol, le dipropylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

De tels solvants organiques peuvent être présents dans des proportions de préférence comprises entre 1 et 40 % en poids par rapport au poids total de chaque composition dans laquelle ils sont contenus, et plus préférentiellement entre 5 et 30 % en poids.

De préférence, les compositions (A) et (B) comprennent de l'eau. De manière plus préférée, chacune des compositions (A) et (B) comprend au moins 5 % en poids d'eau, de préférence au moins 10 % en poids d'eau, et mieux encore au moins 20 % en poids d'eau, par rapport à son poids total.

Les compositions (A) et/ou (B) selon la présente invention peuvent également comprendre un ou plusieurs adjuvants, choisis parmi ceux utilisés classiquement dans les compositions pour la coloration des fibres kératiniques tels que des polymères conditionneurs en particulier cationiques; des agents épaississants; des agents antioxydants différents des réductones; des agents de pénétration ; des agents séquestrants; des parfums ; des agents dispersants ; des agents filmogènes ; des céramides ; des agents conservateurs ; des agents opacifiants.

Les adjuvants ci dessus peuvent être en général présents en quantité comprise pour chacun d'eux entre 0,01 et 20 % en poids par rapport au poids de chaque composition.

Selon un mode de réalisation préféré, la composition (A) se présente sous la forme d'une émulsion huile-dans-eau (dite émulsion directe), ou d'une émulsion eau-dans-huile (dite émulsion inverse).

La présente invention a également pour objet un procédé de coloration des fibres kératiniques, comprenant l'application sur lesdites fibres de l'agent tel que décrit ci-avant.

Selon l'invention, l'agent appliqué sur les fibres kératiniques résulte du mélange des compositions (A) et (B), ce mélange étant réalisé soit avant application sur les fibres kératiniques (préparation extemporanée) soit directement sur les fibres kératiniques (application successive sur les fibres des compositions (A) et (B) sans rinçage intermédiaire).

Ainsi, selon une première variante du procédé selon l'invention, on applique sur les fibres kératiniques, sèches ou humides, successivement et sans rinçage intermédiaire, les compositions (A), puis (B).

Selon une deuxième variante du procédé selon l'invention, on applique sur les fibres kératiniques, sèches ou humides, une composition obtenue par mélange extemporané, avant l'application, des compositions (A) et (B).

Dans ce cas, alors la durée entre le mélange des compositions (A) et (B) et l'application du mélange sur les cheveux n'excède pas de préférence 30 minutes, de préférence 10 minutes, de manière encore préférée 5 minutes.

Indépendamment de la variante mise en oeuvre, le rapport pondéral de la quantité de composition (A) utilisée à la quantité de composition (B) utilisée peut varier de 0,2 à 3 et de préférence de 0,3 à 1.

En outre, indépendamment de la variante mise en oeuvre, le mélange présent sur les fibres (résultant soit du mélange extemporané des compositions (A) et (B) soit de l'application successive de celles-ci) est laissé en place pour une durée, en général, de l'ordre de 1 minute à 1 heure, de préférence de 5 minutes à 30 minutes.

La température durant le procédé est classiquement comprise entre la température ambiante (entre 15 à 25°C) et 80°C, de préférence entre la température ambiante et 60°C.

A l'issue du traitement, les fibres kératiniques sont éventuellement rincées à l'eau, subissent éventuellement un lavage avec un shampooing suivi d'un rinçage à l'eau, avant d'être séchées ou laissées à sécher.

Enfin, l'invention a également pour objet un dispositif à plusieurs compartiments ou « kit » de coloration constitué d'un premier compartiment renfermant une composition (A), et d'un deuxième compartiment renfermant une composition (B), les compositions (A) et (B) étant telles que décrites ci-avant.

Ce dispositif peut avantageusement être équipé d'un moyen permettant de délivrer sur les cheveux le mélange souhaité, tel que les dispositifs décrits dans le brevet FR 2586913.

Ce dispositif peut être accompagné d'une ou plusieurs compositions de lavage et/ou de conditionnement des fibres kératiniques, destiné à être appliquées avant et/ou après le traitement de coloration selon l'invention.

Les exemples suivants servent à illustrer l'invention sans toutefois présenter un caractère limitatif.

### EXEMPLE

Les compositions de coloration d'oxydation suivantes ont été préparées (dans le tableau ci-dessous, les quantités sont exprimées en grammes) :

**Composition colorante (A) :**

| Composition | A1 | A2 |
|---|---|---|
| Huile de vaseline | 55 | 55 |
| Octyl dodécanol | 10 | 10 |
| Hectorite modifiée distéaryl diméthyl ammonium | 1,5 | 1,5 |
| Carbonate de propylène | 0,5 | 0,5 |
| Alcool oléique 10 OE | 5 | 5 |
| Propylène glycol | 2 | 2 |
| Ethanol | 2,5 | 2,5 |
| Hexylène glycol | 1 | 1 |
| Dipropylène glycol | 1 | 1 |
| Monoéthanolamine | 4,5 | 4,5 |
| POE/POP/POE (Poloxamer 184) | 9 | 9 |
| Acide ascorbique | - | 0,25 |
| Para-phénylènediamine | 0,16 | 0,16 |
| Para-aminophénol | 0,12 | 0,12 |
| 5-amino-6-chloro-o-crésol | 0,2 | 0,2 |
| 1-H-pyrazole-1-éthanol,4-5-diamino sulfate | 1,44 | 1,44 |
| 1-méthyl-2-hydrxy-4-amino-benzène | 0,92 | 0,92 |
| Eau | Qsp 100 | Qsp 100 |

La composition A2 correspond à une composition (A) conforme à la présente invention, tandis que la composition A1 est une composition comparative ne contenant pas de réductone.

**Composition oxydante (B) :**

| Composition | B |
|---|---|
| Solution aqueuse à 40% en poids d'acide diéthylene triamine pentacétique, sel pentasodique | 0,375 |
| Solution aqueuse à 50% en poids de peroxyde d'hydrogène | 12 |
| Stannate de sodium | 0,04 |
| Pyrophosphate de tétrasodium | 0,03 |
| Huile de vaseline | 20 |
| Solution aqueuse à 40% en poids de polycondensat tétraméthyl hexaméthylènediamine / dichloro 1,3-propylène ou Hexadimethrine chloride | 0,1 |
| Solution aqueuse à 40% en poids de chlorure de poly diméthyl diallyl ammonium ou Polyquaternium-6, non stabilisé | 0,2 |
| Glycérine | 0,5 |
| Alcool cétéarylique | 8 |
| Alcool cétylstéarylique oxyéthyléné (33 OE) | 3 |
| Amide d'acides de colza oxyéthyléné (4 OE) | 1,2 |
| Vitamine E : DL-α-tocophérol | 0,1 |
| Acide phosphorique | Qs pH 2,2 |
| Eau | Qsp 100 |

Les compositions décrites ci-dessus ont été mélangées au moment de l'emploi, de la manière suivante :
- 10 g de la composition colorante A1 a été mélangé à 10 g de la composition oxydante B d'une part, et
- 10 g de la composition colorante A2 a été mélangé à 10 g de la composition oxydante B d'autre part.

Le mélange résultant des compositions A2 et B se colore moins que le mélange des compositions A1 et B.

Appliqué sur des cheveux, le mélange résultant des compositions A2 et B conduit en outre à des résultats tinctoriaux très homogènes et intenses.

## Revendications

1. Agent de coloration des fibres kératiniques, constitué par:
- une première composition (A) comprenant un ou plusieurs agents alcalinisants, un ou plusieurs colorants d'oxydation, choisis parmi les bases d'oxydation, éventuellement combinée(s) à un ou plusieurs coupleurs, et un ou plusieurs colorants directs, et
- une seconde composition (B) comprenant un ou plusieurs agents oxydants,
l'une au moins des deux compositions (A) et (B) comprenant un ou plusieurs corps gras choisis parmi les hydrocarbures liquides, la quantité totale desdits hydrocarbures liquides dans le mélange des compositions (A) et (B) représentant au moins 20% en poids, par rapport au poids total dudit mélange, et
l'une au moins des deux compositions (A) et (B) comprenant une ou plusieurs réductones.

2. Agent selon la revendication 1, **caractérisé en ce que** l'agent alcalinisant est choisi parmi l'ammoniaque, les carbonates alcalins, les hydroxydes de sodium ou de potassium, les amines organiques telles que par exemple les alcanolamines et leurs dérivés, et les composés de formule (I) suivante : 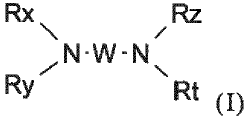 dans laquelle W est un reste alkylène en C₁-C₆ éventuellement substitué par un groupement hydroxyle ou un radical alkyle en C₁-C₆; Rx, Ry, Rz et Rt, identiques ou différents, représentent un atome d'hydrogène, un radical alkyle en C₁-C₆, hydroxyalkyle en C₁-C₆, ou aminoalkyle en C₁-C₆,
et est de préférence choisi parmi les alcanolamines.

3. Agent selon l'une quelconque des revendications précédentes, **caractérisé en ce que** l'agent oxydant est choisi parmi le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux, les enzymes d'oxydo-réduction telles que les laccases, les peroxydases et les oxydoréductases à 2 électrons, éventuellement en présence de leur donneur ou cofacteur respectif; et est de préférence le peroxyde d'hydrogène.

4. Agent selon l'une quelconque des revendications précédentes, **caractérisé en ce que** la ou les réductones sont de formule générale (II) : 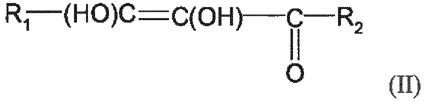 avec R₁ et R₂, identiques ou différents, désignant chacun un groupement contenant au moins un atome de carbone et/ou d'oxygène, R₁ et R₂ pouvant former avec les trois atomes de carbone du composé de formule (II) un cycle de préférence à 5 ou 6 chaînons, dont les atomes constitutifs supplémentaires sont constitués par des atomes de carbone et/ou d'oxygène,
le ou les composés de formule (II) se présentant sous forme acide, ou sous forme de sels, notamment sous forme de sels de métaux alcalins ou de métaux alcalino-terreux, ou sous forme d'esters notamment acides gras en C₈ à C₃₀.

5. Agent selon l'une quelconque des revendications précédentes, **caractérisé en ce que** la ou les réductones sont choisies parmi l'acide ascorbique, l'acide érythorbique, et les sels de ces composés, notamment les sels de sodium et de potassium.

6. Agent selon l'une quelconque des revendications précédentes, **caractérisé en ce que** la quantité de réductone(s) dans la composition (A) et/ou la composition (B) représente de 0,01 à 1% en poids, de préférence de 0,05 à 0,5% en poids, et mieux encore de 0,1 à 0,25% en poids, par rapport au poids total de la composition (A) et/ou respectivement de la composition (B), ces pourcentages en poids étant exprimés par rapport à la forme acide de la ou des réductones.

7. Agent selon l'une quelconque des revendications précédentes, **caractérisé en ce que** la quantité totale de réductone(s) dans le mélange des compositions (A) et (B) représente de 0,01 à 1% en poids, de préférence de 0,05 à 0,5% en poids, et mieux encore de 0,1 à 0,2% en poids, par rapport au poids total dudit mélange, ces pourcentages en poids étant exprimés par rapport à la forme acide de la ou des réductones.

8. Agent selon l'une quelconque des revendications précédentes, **caractérisé en ce que** ledit ou lesdits hydrocarbures liquides sont choisis parmi :
- les alcanes inférieurs en C₆-C₁₆ linéaires ou ramifiés, éventuellement cycliques, et
- les hydrocarbures linéaires ou ramifiés, d'origine minérale animale ou synthétique, contenant plus de 16 atomes de carbone,
et de préférence sont choisis parmi les huiles de paraffine, l'huile de vaseline, et leurs mélanges.

9. Agent selon l'une quelconque des revendications précédentes, **caractérisé en ce que** la quantité totale desdits hydrocarbures liquides dans le mélange des compositions (A) et (B) représente au moins 25% en poids, et de préférence au moins 30% en poids, par rapport au poids total dudit mélange.

10. Agent selon l'une quelconque des revendications précédentes, **caractérisé en ce que** la composition (A) et/ou la composition (B) comprend un ou plusieurs corps gras additionnels, différents desdits hydrocarbures liquides, et qui ne contiennent pas de fonction acide carboxylique, de préférence choisis parmi les huiles non siliconées d'origine végétale ou synthétique, les alcools gras, les esters d'acide gras et/ou d'alcool gras, les cires non siliconées, et les silicones.

11. Agent selon l'une quelconque des revendications précédentes, **caractérisé en ce que** la composition (A) se présente sous la forme d'une émulsion huile-dans-eau, ou d'une émulsion eau-dans-huile.

12. Procédé de coloration des fibres kératiniques, comprenant l'application sur lesdites fibres, sèches ou humides, successivement et sans rinçage intermédiaire, des compositions (A) puis (B) telles que définies dans l'une quelconque des revendications précédentes.

13. Procédé de coloration des fibres kératiniques, comprenant l'application sur lesdites fibres, sèches ou humides, d'une composition obtenue par mélange extemporané des compositions (A) et (B) telles que définies dans l'une quelconque des revendications 1 à 11.

14. Dispositif à plusieurs compartiments ou « kit » de coloration, constitué d'un premier compartiment renfermant une composition (A), et d'un deuxième compartiment renfermant une composition (B), les compositions (A) et (B) étant telles que définies dans l'une quelconque des revendications 1 à 11.

## Patentansprüche

1. Mittel zum Färben von Keratinfasern, bestehend aus :
- einer ersten Zusammensetzung (A), die ein oder mehrere Alkalinisierungsmittel, einen oder mehrere Oxidationsfarbstoffe, die aus Oxidationsbasen, gegebenenfalls in Kombination mit einem oder mehreren Kupplern, ausgewählt sind, und einen oder mehrere Direktfarbstoffe umfasst, und
- einer zweiten Zusammensetzung (B), die ein oder mehrere Oxidationsmittel umfasst,
wobei mindestens eine der beiden Zusammensetzungen (A) und (B) eine oder mehrere Fettsubstanzen, die aus flüssigen Kohlenwasserstoffen ausgewählt sind, umfasst, wobei die Gesamtmenge der flüssigen Kohlenwasserstoffe in der Mischung der Zusammensetzungen (A) und (B) mindestens 20 Gew.-%, bezogen auf das Gesamtgewicht der Mischung, ausmacht, und
mindestens eine der beiden Zusammensetzungen (A) und (B) ein oder mehrere Reduktone umfasst.

2. Mittel nach Anspruch 1, **dadurch gekennzeichnet, dass** das Alkalinisierungsmittel unter wässrigem Ammoniak, Alkalimetallcarbonaten, Natrium- oder Kaliumhydroxid, organischen Aminen wie beispielsweise Alkanolaminen und deren Derivaten und den Verbindungen der folgenden Formel (I) ausgewählt ist: 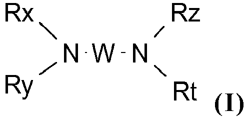
worin W für einen C₁-C₆-Alkylenrest, der gegebenenfalls durch eine Hydroxylgruppe oder einen C₁-C₆-Alkylrest substituiert ist, steht;
Rx, Ry, Rz und Rt gleich oder verschieden sind und für ein Wasserstoffatom, einen C₁-C₆-Alkylrest, einen C₁-C₆-Hydroxyalkylrest oder einen C₁-C₆-Aminoalkylrest stehen;
und vorzugsweise unter Alkanolaminen ausgewählt ist.

3. Mittel nach einem der vorhergehenden Ansprüche, **dadurch gekennzeichnet, dass** das Oxidationsmittel unter Wasserstoffperoxid, Harnstoffperoxid, Alkalimetallbromaten oder -ferricyaniden, Peroxysalzen wie beispielsweise Alkalimetall- oder Erdalkalimetallpersulfaten, -perboraten und -percarbonaten, Redoxenzymen wie Laccasen, Peroxidasen und 2-Elektronen-Oxidoreduktasen, gegebenenfalls in Gegenwart des jeweiligen Donors oder Cofaktors davon, ausgewählt ist und vorzugsweise Wasserstoffperoxid ist.

4. Mittel nach einem der vorhergehenden Ansprüche, **dadurch gekennzeichnet, dass** das Redukton bzw. die Reduktone die allgemeine Formel (II) aufweist bzw. aufweisen: 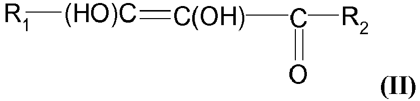 wobei R₁ und R₂ gleich oder verschieden sind und jeweils für eine Gruppe, die mindestens ein Kohlenstoff- und/oder Sauerstoffatom enthält, stehen, wobei R₁ und R₂ mit den drei Kohlenstoffatomen der Verbindung der Formel (II) einen vorzugsweise 5- oder 6-gliedrigen Ring bilden können, dessen zusätzliche Atombausteine aus Kohlenstoff- und/oder Sauerstoffatomen bestehen,
wobei die Verbindung bzw. die Verbindungen der Formel (II) in Säureform oder in Form von Salzen, insbesondere in Form von Alkalimetall- oder Erdalkalimetallsalzen, oder in Form von Estern, insbesondere von C₈-bis C₃₀-Fettsäuren, vorliegt bzw. vorliegen.

5. Mittel nach einem der vorhergehenden Ansprüche, **dadurch gekennzeichnet, dass** das Redukton bzw. die Reduktone aus Ascorbinsäure, Erythorbinsäure und Salzen dieser Verbindungen, insbesondere Natrium-und Kaliumsalzen, ausgewählt ist bzw. sind.

6. Mittel nach einem der vorhergehenden Ansprüche, **dadurch gekennzeichnet, dass** die Menge an Redukton bzw. Reduktonen in der Zusammensetzung (A) und/oder der Zusammensetzung (B) 0,01 bis 1 Gew.-%, vorzugsweise 0,05 bis 0,5 Gew.-% und noch besser 0,1 bis 0,25 Gew.-%, bezogen auf das Gesamtgewicht der Zusammensetzung (A) und/oder respektive der Zusammensetzung (B), beträgt, wobei sich diese Gewichtsprozentangaben auf die Säureform des Reduktons bzw. der Reduktone beziehen.

7. Mittel nach einem der vorhergehenden Ansprüche, **dadurch gekennzeichnet, dass** die Menge an Redukton bzw. Reduktonen in der Mischung der Zusammensetzungen (A) und (B) 0,01 bis 1 Gew.-%, vorzugsweise 0,05 bis 0,5 Gew.-% und noch besser 0,1 bis 0,2 Gew.-%, bezogen auf das Gesamtgewicht der Mischung, beträgt, wobei sich diese Gewichtsprozentangaben auf die Säureform des Reduktons bzw. der Reduktone beziehen.

8. Mittel nach einem der vorhergehenden Ansprüche, **dadurch gekennzeichnet, dass** der flüssige Kohlenwasserstoff bzw. die flüssigen Kohlenwasserstoffe aus
- linearen oder verzweigten, gegebenenfalls cyclischen C₆-C₁₆-Niederalkanen und
- linearen oder verzweigten Kohlenwasserstoffen mineralischer, tierischer oder synthetischer Herkunft, die mehr als 16 Kohlenstoffatome enthalten,
ausgewählt ist bzw. sind und vorzugsweise aus Paraffinölen, Vaselineöl und Mischungen davon ausgewählt ist bzw. sind.

9. Mittel nach einem der vorhergehenden Ansprüche, **dadurch gekennzeichnet, dass** die Gesamtmenge an flüssigen Kohlenwasserstoffen in der Mischung der Zusammensetzungen (A) und (B) mindestens 25 Gew.-% und vorzugsweise mindestens 30 Gew.-%, bezogen auf das Gesamtgewicht der Mischung, beträgt.

10. Mittel nach einem der vorhergehenden Ansprüche, **dadurch gekennzeichnet, dass** die Zusammensetzung (A) und/oder die Zusammensetzung (B) eine oder mehrere zusätzliche Fettsubstanzen, die von den flüssigen Kohlenwasserstoffen verschieden sind und keine Carbonsäurefunktion enthalten, und vorzugsweise aus Nichtsilikonölen pflanzlicher oder synthetischer Herkunft, Fettalkoholen, Fettsäureestern und/oder Fettalkoholestern, Nichtsilikonwachsen und Silikonen ausgewählt sind, umfasst bzw. umfassen.

11. Mittel nach einem der vorhergehenden Ansprüche, **dadurch gekennzeichnet, dass** die Zusammensetzung (A) in Form einer Öl-in-Wasser-Emulsion oder einer Wasser-in-Öl-Emulsion vorliegt.

12. Verfahren zum Färben von Keratinfasern, bei dem man auf die trockenen oder feuchten Fasern nacheinander und ohne Zwischenspülung die Zusammensetzungen (A) und dann (B) gemäß einem der vorhergehenden Ansprüche aufbringt.

13. Verfahren zum Färben von Keratinfasern, bei dem man auf die trockenen oder feuchten Fasern eine durch unmittelbar vorhergehendes Mischen der Zusammensetzungen (A) und (B) gemäß einem der Ansprüche 1 bis 11 erhaltene Zusammensetzung aufbringt.

14. Vorrichtung mit mehreren Kompartimenten oder "Kit" zum Färben, bestehend aus einem ersten Kompartiment, das eine Zusammensetzung (A) enthält, und einem zweiten Kompartiment, das eine Zusammensetzung (B) enthält, wobei die Zusammen-setzungen (A) und (B) wie in einem der Ansprüche 1 bis 11 definiert sind.

## Claims

1. Agent for colouring keratin fibres, consisting of:
- a first composition (A) comprising one or more alkalizing agents, one or more oxidation dyes, selected from oxidation bases, optionally combined with one or more couplers, and one or more direct dyes, and
- a second composition (B) comprising one or more oxidizing agents,
at least one of the two compositions (A) and (B) comprising one or more fats selected from the liquid hydrocarbons, the total amount of said liquid hydrocarbons in the mixture of compositions (A) and (B) representing at least 20 wt.%, relative to the total weight of said mixture, and
at least one of the two compositions (A) and (B) comprising one or more reductones.

2. Agent according to Claim 1, **characterized in that** the alkalizing agent is selected from ammonia, alkaline carbonates, sodium or potassium hydroxide, organic amines such as for example alkanolamines and their derivatives, and the compounds of the following formula (I): 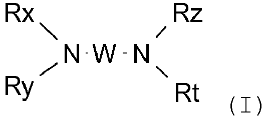 in which W is a C₁-C₆ alkylene residue optionally substituted with a hydroxyl group or a C₁-C₆ alkyl radical; Rx, Ry, Rz and Rt, which may be identical or different, represent a hydrogen atom, a C₁-C₆ alkyl, C₁-C₆ hydroxyalkyl, or C₁-C₆ aminoalkyl radical,
and is preferably selected from the alkanolamines.

3. Agent according to either one of the preceding claims, **characterized in that** the oxidizing agent is selected from hydrogen peroxide, urea peroxide, bromates or ferricyanides of alkali metals, peroxidized salts for example persulfates, perborates and percarbonates of alkali metals or alkaline-earth metals, redox enzymes such as laccases, peroxidases and 2-electron oxidoreductases, optionally in the presence of their respective donor or cofactor; and is preferably hydrogen peroxide.

4. Agent according to any one of the preceding claims, **characterized in that** the reductone or reductones are of general formula (II): 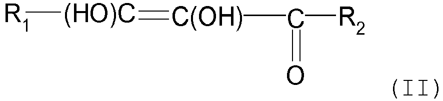 with R₁ and R₂, which may be identical or different, each denoting a group containing at least one carbon atom and/or oxygen atom, and R₁ and R₂ can form, with the three carbon atoms of the compound of formula (II), a ring preferably with 5 or 6 ring members, whose additional constituent atoms are constituted of carbon atoms and/or oxygen atoms,
the compound or compounds of formula (II) being in the form of acid, or in the form of salts, notably in the form of salts of alkali metals or of alkaline-earth metals, or in the form of esters notably of C₈ to C₃₀ fatty acids.

5. Agent according to any one of the preceding claims, **characterized in that** the reductone or reductones are selected from ascorbic acid, erythorbic acid, and the salts of these compounds, notably the sodium and potassium salts.

6. Agent according to any one of the preceding claims, **characterized in that** the amount of reductone(s) in composition (A) and/or composition (B) represents from 0.01 to 1 wt.%, preferably from 0.05 to 0.5 wt.%, and better still from 0.1 to 0.25 wt.%, relative to the total weight of composition (A) and/or respectively of composition (B), these percentages by weight being expressed relative to the acid form of the reductone or reductones.

7. Agent according to any one of the preceding claims, **characterized in that** the total amount of reductone(s) in the mixture of compositions (A) and (B) represents from 0.01 to 1 wt.%, preferably from 0.05 to 0.5 wt.%, and better still from 0.1 to 0.2 wt.%, relative to the total weight of said mixture, these percentages by weight being expressed relative to the acid form of the reductone or reductones.

8. Agent according to any one of the preceding claims, **characterized in that** said liquid hydrocarbon or said liquid hydrocarbons are selected from:
- linear or branched, optionally cyclic, C₆-C₁₆ lower alkanes and
- linear or branched hydrocarbons, of mineral, animal or synthetic origin, containing more than 16 carbon atoms,
and preferably are selected from paraffin oils, liquid paraffin, and mixtures thereof.

9. Agent according to any one of the preceding claims, **characterized in that** the total amount of said liquid hydrocarbons in the mixture of compositions (A) and (B) represents at least 25 wt.%, and preferably at least 30 wt.%, relative to the total weight of said mixture.

10. Agent according to any one of the preceding claims, **characterized in that** composition (A) and/or composition (B) comprises one or more additional fats, different from said liquid hydrocarbons, and which do not contain a carboxylic acid function, preferably selected from non-silicone oils of vegetable or synthetic origin, fatty alcohols, esters of fatty acid and/or of fatty alcohol, non-silicone waxes, and silicones.

11. Agent according to any one of the preceding claims, **characterized in that** composition (A) is in the form of an oil-in-water emulsion, or of a water-in-oil emulsion.

12. Method of colouring keratin fibres, comprising the application of compositions (A) and then (B) as defined in any one of the preceding claims on said fibres, dry or wet, successively and without intermediate rinsing.

13. Method of colouring keratin fibres, comprising the application of a composition, obtained by extemporaneous mixing of compositions (A) and (B) as defined in any one of Claims 1 to 11, on said fibres, dry or wet.

14. Multi-compartment device or "kit" for colouring, constituted of a first compartment containing a composition (A), and of a second compartment containing a composition (B), compositions (A) and (B) being as defined in any one of Claims 1 to 11.
